# EUROPEAN PATENT SPECIFICATION

(11) **EP 4 316 535 B1**
(45) Date of publication and mention of the grant of the patent: **20.11.2024**
(21) Application number: 22188230.1
(22) Date of filing: 02.08.2022
(51) Int. Cl.: A61L 15/28, A61L 15/40, A61L 15/44, A61K 9/00

(54) **WOUND HEALING COMPOSITION CONTAINING SNAIL MUCUS**
WUNDHEILUNGSZUSAMMENSETZUNG, DIE SCHNECKENSCHLEIM ENTHÄLT
COMPOSITION DE CICATRISATION DE PLAIES CONTENANT DU MUCUS D'ESCARGOT

(43) Date of publication of application: 07.02.2024
(73) Proprietor: Glavash Dodov, Marija, 1000 Skopje (MK); Polenakovikj, Radmil, 1000 Skopje (MK); Simonoska Crcarevska, Maja, 1000 Skopje (MK); Jovanovski R., Bojan, 8010 Graz (AT); Velkovski, Trajche, 1000 Skopje (MK)
(72) Inventor: Glavash Dodov, Marija, 1000 Skopje (MK); Polenakovikj, Radmil, 1000 Skopje (MK); Simonoska Crcarevska, Maja, 1000 Skopje (MK); Jovanovski R., Bojan, 8010 Graz (AT); Velkovski, Trajche, 1000 Skopje (MK)
(74) Representative: Ilievski, Bogoljub

(56) References cited:
- WO-A1-2019/155389
- WO-A1-2020/144564
- WO-A1-2020/201439
- US-B2- 9 125 964
- DATABASE WPI Week 2020006, Derwent World Patents Index; AN 2020-75300Q, XP002808415

## Description

### TECHNICAL FIELD

The present invention belongs to the field of wound healing. More specifically, the present invention discloses a wound healing composition comprising natural raw materials, such as snail mucus and herbal extracts, as active ingredients. In addition, the present application discloses a method for producing the wound healing composition, in the micro-particle form.

### BACKGROUND OF INVENTION

Chronic wounds represent a substantial burden on healthcare systems as incidences are rising each year in developed countries. In the EU alone, the annual incidence of chronic wounds is estimated at four million individuals, and in total around 4% of health expenditure is allocated to chronic wound treatment. The current therapy regimes linked to infected wounds are relatively long-term, including treatment with skin substitutes, negative pressure wound therapy, growth factors and hyperbaric oxygen, in addition to wound dressings and care (several times daily). Prolonged frequent use of conventional pharmacological active substances, such as antibiotics, can also lead to systemic toxicity and antibiotic resistance. Therefore, effective and targeted treatment of wounds is needed in order to improve patient outcomes and decrease the economic burden of such conditions.

In recent years, the mucus or slime produced by snails has been extensively researched based on findings of its effective application in dermatology and cosmetics industries. Notably, the composition of snail mucus (mainly allantoin, collagen, elastin and glycolic acid) has been shown to improve skin condition, likely due to its high protein content and significant effects on cell regeneration and growth, whilst inhibiting inflammatory processes. However, its applications in the wound healing process have not been explored yet.

Pharmaceutical compositions, films or bio-gels, and/or methods for preparing such products, for prevention and/or treatment of wounds and/or skin conditions are well documented in both patent and scientific literature, but these are all associated with disadvantages compared to the present invention.

US9125964B2 describes a pharmaceutical composition and a method for treating a diabetic foot wound. The composition described is a viscous formulation that can be incorporated into conventional gels or creams and soaked in gauze and comprises snail slime and marigold extract.

IN202041030334 describes a method for preparing a bio-gel synthesized from a mixture of snail slime and chitosan in powder form, wherein the chitosan is obtained from deacetylation of chitin. The resulting bio-gel is used for surgical wound healing.

WO2019/155389A1 describes a composition for the prevention and treatment of ulcerative, inflammatory and/or erosive disorders of mucous membranes. The composition comprises: a mucoadhesive agent (such as chitin or chitosan), a bioadhesive agent (such as snail mucus), a pharmaceutical agent, a natural polymer (such as chitin or chitosan), and a cannabinoid (such as CBD, CBG or CBN). Furthermore, the composition may additionally comprise an anti-inflammatory agent such as Calendula.

WO2020/201439A1 describes a film comprising gastropod slime such as snail slime or mucus and a polymer, such as chitosan, that is used for the treatment of various skin conditions, such as atopic dermatitis and psoriasis. The film may additionally comprise a microparticle system to modulate the release of the composition and increase skin permeability and its passage through the skin barrier.

WO2020/144564A1 describes a composition comprising allantoin (extracted from snail slime or mucus), *Hypericum* oil (extracted from St. John's-wort) and *Calendula.* The composition may be formulated as a spray for the treatment of various skin conditions, such as psoriasis and eczema.

### SUMMARY OF INVENTION

The scope of protection is defined by the claims.

The present application discloses a complex microparticulated system containing snail slime, chitosan and herbal extracts, that provides multi target synergistic approach, which is particularly suitable as a final dosage formulation for wound treatment. The purpose of this approach is to avoid the use of conventional pharmacological active substances, such as antibiotics, for wound treatment. Thus, the present invention utilizes a multicomponent approach to achieve the desired treatment.

Thus, the present application discloses a novel approach for wound healing, based on the use of snail mucus in combination with other natural raw materials as active substances. The natural raw materials include chitosan, and herbal extracts of cannabis, St. John's-wort *(Hypericum perforatum)* and pot marigold *(Calendula officinalis).* These components are embedded in one step process into microparticles and can be incorporated into an appropriate dosage form, such as a dressing, gel, cream, lotion, topical powder etc.

The composition according to the present invention contains selected herbal extracts as well as snail slime, which are embedded into chitosan-based microparticles, and preferably, subsequently formulated into film-dressing. Chitosan-based microparticles containing embedded active ingredients provide adequate swelling properties and prolonged release of active ingredients, thus enabling better therapeutic efficacy and wound treatment outcome.

The present invention proposes the composition containing snail slime, Cannabidiol (CBD), St. John's-wort extract and pot marigold extract, embedded into chitosan-based microparticles. The proposed combination, and particularly formulation based on active ingredients embedded in chitosan-based microparticles, contributes to enhanced antioxidant, anti-inflammatory, antimicrobial, and wound healing acceleration properties, without the risk of systemic toxicity, antibiotic resistance and/or other negative side effects associated with conventional treatments. Furthermore, the microparticles containing the composition according to the present invention incorporated in topical formulations, such as film-dressing, gel, cream, lotion, or topical powder, enable easy application, with controlled and extended-release properties, thus prolonging dosage intervals (once every couple of days), whilst enhancing efficacy in wound healing.

In one aspect, the present invention provides a wound healing composition according to claim comprising: Snail mucus, chitosan, Cannabidiol (which may be in the form of CBD oil which is made by ethanolic extraction, or CBD extract obtained by CO2 extraction, or CBD crystals (isolates) from the cannabis plant and then diluted with a carrier oil like coconut oil, hemp seed oil or medium chain triglycerides), and ethanolic extracts of St. John's-wort *(Hypericum perforatum)* and pot marigold *(Calendula officinalis*).

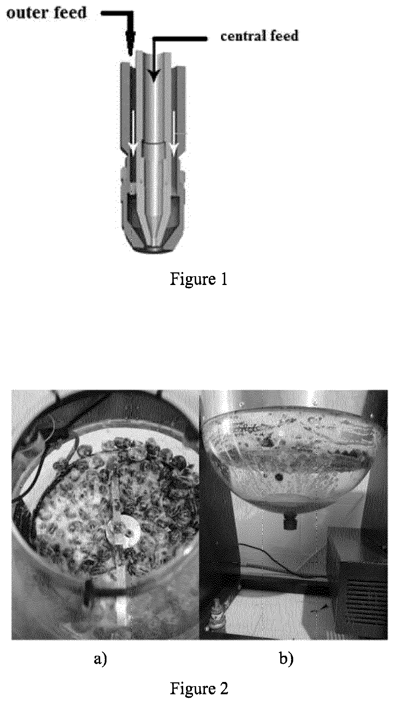

In another aspect, the present invention provides a method for making the wound healing composition, the method comprising the steps of:
combining snail mucus, chitosan, Cannabidiol in form of CBD oil, and ethanolic extracts of St. John's-wort *(Hypericum perforatum)* and pot marigold *(Calendula officinalis);* and
formation of microparticles, preferably by spray drying technique, preferably by using double fluid nozzle, where snail mucus, Cannabidiol in form of CBD oil and ethanolic extracts of St. John's-wort *(Hypericum perforatum)* and pot marigold *(Calendula officinalis) and* cross-linking agent, such as tripolyphosphate (TPP), are sprayed through the central feed, while chitosan solution i.e. chitosan dissolved in 1% glacial acetic acid or 1% hydrochloric acid, is sprayed through the outer feed of the double fluid nozzle (Fig. 1), which results in formation of microparticles. The composition undergoes spray-drying at elevated temperatures, preferably at 150° C.

The present invention therefore provides a natural approach to wound healing, with improved efficacy, reduced or minimal side effects in comparison to conventional pharmacologically active substances, such as antibiotics, while at the same time allows easy application with controlled and extended-release properties, thus prolonging dosage intervals (once every couple of days).

### BRIEF DESCRIPTION OF DRAWINGS:

Fig. 1. Schematic representation of double fluid nozzle.
Fig. 2a: represents the top view of the snail mucus extractor.
Fig. 2b: represents the bottom view of the collection vessel of snail mucus extractor.
Fig. 3: Macroscopic (left) and microscopic (right) images of snail mucus powder.
Fig. 4: illustrates an overview of the key components of the composition according to the present invention.
Fig. 5: is a schematic representation of microparticles (107) containing incorporated active ingredients according to the present invention.
Fig. 6a-6c: represents microscopic images of spray-dried swellable microparticles of the present invention containing the snail mucus: a) image under light microscope b) TEM (transmission emission microscope) image c) zoomed TEM image.

### DETAILED DESCRIPTION

Disclosed in the present application is a wound healing composition containing natural raw materials, Snail mucus, *Cannabis sativa, Hypericum perforatum and Calendula officinals* as active substances, incorporated into a biopolymer network, chitosan cross-linked with e.g., TPP.

The composition according to the present invention comprises the following components: snail mucus, chitosan, oil extract of Cannabis sativa (particularly the active ingredient Cannabidiol), as well as ethanolic extracts of St. John's-wort and pot marigold.

Due to its specific composition and multifunctional properties (wound healing, preventing or treating infections, scar reducing, etc.), the present invention finds particular application in the following fields: biomedical applications in humans and animals, such as for the treatment of skin conditions or wounds; and cosmetic applications in humans, such as anti-aging or acne treatment. Aside from the aforementioned benefits of snail mucus in improving skin condition, the introduction of chitosan and herbal extracts to the wound healing composition provides additional advantages. Chitosan is a naturally occurring marine polysaccharide derived from the deacetylation of chitin, which serves as a polymer matrix and supports tissue growth by stimulating cell proliferation. Cannabis sativa extracts are used due to their antioxidant and anti-inflammatory activities arising from biologically active compounds, such as amino acids, fatty acids, steroids, flavonoids, lignans, terpenoids, alkaloids and cannabinoids (notably cannabidiol (CBD), cannabinol (CBN) and cannabigerol (CBG)). Similarly, the clinical rationale for use of St. John's- wort ethanolic extract in the management of wounds is due to its antimicrobial and anti-inflammatory activities, as well as stimulatory effects on fibroblast motility, collagen production, and keratinocyte differentiation. Finally, pot marigold extract is included due to its anti-inflammatory, antimicrobial, antioxidant, and wound healing acceleration activities.

In one embodiment of the invention, the snail mucus extract is obtained using the following method, comprising two phases. In the first phase, freshly harvested snails, preferably, 2.5 kg, are gathered and then rinsed with warm distilled water at about 30-35°C, for removal of environmental and mechanical contaminants. Any type of snails can be used for this purpose. Said rinsing cycle is repeated three times. The snails are subsequently transferred to an extractor which is operably connected to the collection vessel for collecting the extracted snail mucus. The extractor preferably runs for at least 1 hour at about 30 rpm (see Figure 2a). In the 20^{th} and 40^{th} minute of the 1hr of extraction, 200 ml of distilled water is sprayed on the snails in order to dilute the extracted snail mucus for easier flowing into the collection vessel. After about one hour, the rotation is stopped, and 200 ml of distilled water is sprayed on the processed snails three times. Between each round of water spraying the snails in the extractor, the snails are inverted i.e.mixed. The snail mucus extract located in the lower part of the collection vessel, which is operably connected to the extractor, is strained through a sieve (Fig. 2b). Subsequently, the obtained liquid snail mucus extract is transferred to a second collection vessel.

In the second phase, distilled water, preferably IL, is added to the processed snails from the first phase and mixed, preferably for about 10 minutes, to obtain the residue of the snail mucus. Said mucus residue is then separated from the processed snails by means of three consecutive separation steps of the phase two using sieves of different mesh size openings (from larger to smaller). The resulting residue mucus is mixed with the mucus collected from the first phase and filtered through filter under vacuum, preferably sintered glass 11G3 filter under vacuum. Optionally, the mucus may be diluted with distilled water in mucus:water ratio of max 1:4.

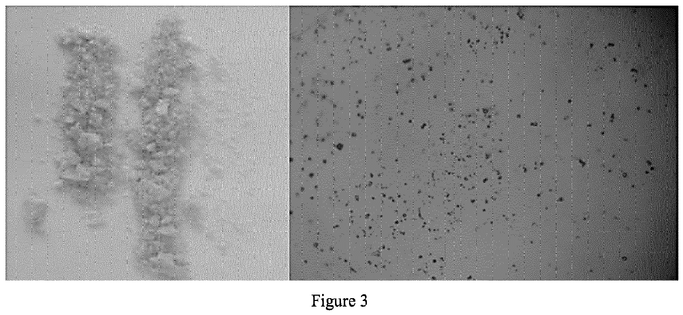

The liquid mucus is freeze dried at -45 °C for at least 24 hours until a dry powder is obtained (see Figure 3). Freeze drying is a low temperature dehydration process which involves freezing the mucus, reducing the pressure and adding heat to remove any ice by transforming it into water vapor (sublimation).

Preferably, the snail mucus is in the form of powder. More preferably, the powder has a particle size of D50 4.7µm±0.0035 with Span value of 1.48±0.006. The said powder particle size D50 and Span value are determined in 96% ethanol by laser diffractometry using Mastersizer 2000, Malvern Instruments Ltd., UK equipped with Hydro 2000S, Malvern Instruments Ltd., UK, for wet dispersions.

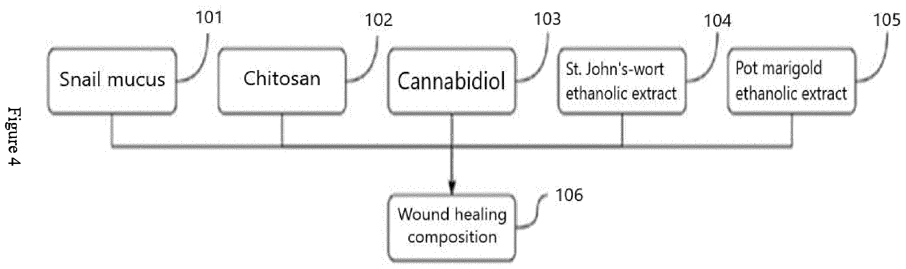

Figure 4 provides an overview of the key components of the present invention that are combined to form the wound healing composition (106). The key components of the wound healing composition (106) are snail mucus extract (101), chitosan (102), Cannabidiol in form of CBD oil (103), St. John's-wort ethanolic extract (104) which preferably is prepared with 45% ethanol where the ratio between St. John's-wort and ethanol is 1:10 (w/w), and pot marigold ethanolic extract (105) which preferably is prepared with 70% ethanol where the ratio between pot marigold and ethanol is 1:10 (w/w). The w/w stands for weight per weight.

Thus, the wound healing composition (106) according to the present invention comprises the following active ingredients: snail mucus (101), Cannabidiol in form of CBD oil (103), *Hypericum perforatum* ethanolic extract *(104)* and *Calendula officinalis* ethanolic extract (105) which are embedded chitosan-based (102) microparticles.

In a preferred embodiment of the invention, the wound healing composition (106) comprises:
Snail mucus, 10-50 wt%;
Chitosan, 10-15 wt%;
Cannabidiol, 0.01-0.5 wt%;
*Hypericum perforatum* ethanolic extract, 0.1-1 wt%;
*Calendula officinalis* ethanolic extract, 0.1-1 wt% and
Cross linking agent, preferably,
Tripolyphosphate 35-40 wt%,
based on the total weight of the composition.

More preferably, the wound healing composition comprises:
Snail mucus, 49.5 wt%;
Chitosan, 12.45 wt%;
Cannabidiol, 0.05 wt%;
*Hypericum perforatum* ethanolic extract, 0.5 wt%;
*Calendula officinalis* ethanolic extract, 0.5 wt% and
Tripolyphosphate 37 wt%,
based on the total weight of the composition.

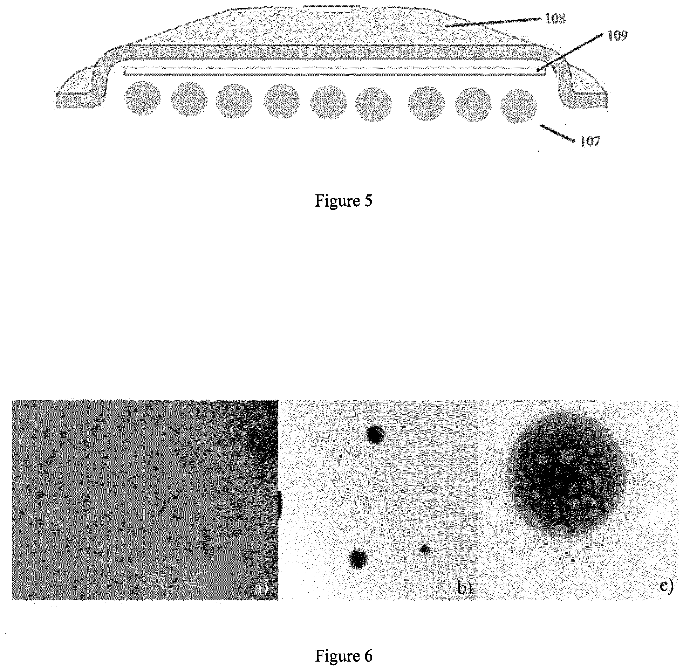

The wound healing composition is in the form of chitosan-based microparticles (107) as shown in Figure 5. The microparticles are subsequently incorporated into an appropriate dosage form, such as a dressing (Figure 5) composed of backing adhesive (108) and film (109) loaded with microparticles (107), cream, gel, ointment, lotion, etc. Incorporation can be achieved using known methods in the art, for example through the use of an adhesive.

In a preferred embodiment of the invention, a spray drying technique is used to produce the chitosan-based microparticles (see Figure 6). Spray drying is a continuous process for transforming a liquid or slurry feed into a powder (dried particles) by means of a gaseous hot drying medium. The resulting microparticles contain the active ingredients embedded. The weight percentage of snail mucus embedded into microparticles, expressed as total protein content, is ≥15%, as

determined by biuret test for protein (Cary 60 UV/VIS spectrophotometer, Agilent, USA). The Cannabidiol in the form of CBD oil, St. John's-wort ethanolic extract and Pot marigold ethanolic extract embedded into microparticles, expressed as total phenols is ≥0.7 wt%. The microparticle size (D50) is in range of 5-7 µm with Span value in range of 1-2.5 when determined in 96% ethanol by laser diffractometry using Mastersizer 2000, Malvern Instruments Ltd., UK equipped with Hydro 2000S, Malvern Instruments Ltd., UK, for wet dispersions. Surface zeta potential of microparticles is in range of -25 to -45 mV determined in distilled water using Zetasizer Nano Series, Nano-ZS, Malvern Instruments Ltd., UK.

As well known in the art, this technique offers retention of bioactivity, efficacy and stability of all active ingredients present.

## Claims

1. A wound healing composition comprising the following components:
• Snail mucus;
• Chitosan;
• Cannabidiol;
• *Hypericum perforatum* ethanolic extract and
• *Calendula officinalis* ethanolic extract
**characterized by**
the components being embedded with a cross linking agent in chitosan-based microparticles.

2. The wound healing composition according to claim 1, **characterized in that** the snail mucus is in a powder form.

3. The wound healing composition according to claim 2, **characterized in that** the snail mucus powder has a particle size of D50 4.7µm±0.0035 with Span value of 1.48±0.006.

4. The wound healing composition according to claims 1 to 3, **characterized in that** Cannabidiol is in form of CBD oil.

5. The wound healing composition according to claims 1 to 4, **characterized in that** the composition contains snail mucus 10-50 wt%, based on the total weight of the composition.

6. The wound healing composition according to claims 1 to 5, **characterized in that** the composition contains chitosan 10-15 wt%, based on the total weight of the composition.

7. The wound healing composition according to claims 1 to 6, **characterized in that** the composition contains Cannabidiol 0.01-0.5 wt%, based on the total weight of the composition.

8. The wound healing composition according to claims 1 to 7, **characterized in that** the composition contains *Hypericum perforatum* ethanolic extract 0.1-1 wt%, based on the total weight of the composition.

9. The wound healing composition according to claims 1 to 8, **characterized in that** the composition contains *Calendula officinalis* ethanolic extract 0.1-1 wt%, based on the total weight of the composition.

10. The wound healing composition according to claims 1 to 9, **characterized in that** the composition contains:
Snail mucus, 49.5 wt%;
Chitosan, 12.45 wt%;
Cannabidiol, 0.05 wt%;
*Hypericum perforatum* ethanolic extract, 0.5 wt% and *Calendula officinalis* ethanolic extract,
0.5wt%, based on the total weight of the composition.

11. A topical drug formulation for wound healing containing the wound healing composition according to claims 1-10.

12. The topical drug formulation according to claim 11, **characterized in that** the formulation is in the form of a film, cream, gel, ointment or a lotion.

13. A method of making the wound healing composition according to claims 1-10, the method comprising the steps of:
- extraction of snail mucus from snails;
- freeze drying of snail mucus extract obtained in previous step, for at least 24 hours, at temperature at -45°C to obtain dry snail mucus powder;
- combining the snail mucus obtained in the previous step with Cannabidiol, *Hypericum perforatum* ethanolic extract and *Calendula officinalis* ethanolic extract and with a cross linking agent; and
- embedding into chitosan-based microparticles preferably by spray-drying process using double-fluid nozzle.

14. The wound healing composition according to claims 1 to 10 for use as a medicament.

15. The wound healing composition according to claims 1-10 for use in treatment of skin condition or wound, such as psoriasis, eczema, atopic dermatitis, diabetic foot ulcer.

## Patentansprüche

1. Eine Wundheilungszusammensetzung, die die folgenden Komponenten umfasst:
• Schneckenschleim;
• Chitosan;
• Cannabidiol;
• Ethanolischer Extrakt aus Hypericum perforatum und
• Ethanolischer Extrakt aus Calendula officinalis,
**dadurch gekennzeichnet, dass**
die Komponenten mit einem Vernetzungsmittel in Mikropartikel auf Chitosanbasis eingebettet sind.

2. Die Wundheilungszusammensetzung nach Anspruch 1, **dadurch gekennzeichnet, dass** der Schneckenschleim in Pulverform vorliegt.

3. Die Wundheilungszusammensetzung nach Anspruch 2, **dadurch gekennzeichnet, dass** das Schneckenschleimpulver eine Partikelgröße von D50 4,7 µm ± 0,0035 mit einem Span-Wert von 1,48 ± 0,006 aufweist.

4. Die Wundheilungszusammensetzung nach den Ansprüchen 1 bis 3, **dadurch gekennzeichnet, dass** Cannabidiol in Form von CBD-Öl vorliegt.

5. Die Wundheilungszusammensetzung nach den Ansprüchen 1 bis 4, **dadurch gekennzeichnet, dass** die Zusammensetzung Schneckenschleim in einer Menge von 10-50 Gew.-% enthält, bezogen auf das Gesamtgewicht der Zusammensetzung.

6. Die Wundheilungszusammensetzung nach den Ansprüchen 1 bis 5, **dadurch gekennzeichnet, dass** die Zusammensetzung Chitosan in einer Menge von 10-15 Gew.-% enthält, bezogen auf das Gesamtgewicht der Zusammensetzung.

7. Die Wundheilungszusammensetzung nach den Ansprüchen 1 bis 6, **dadurch gekennzeichnet, dass** die Zusammensetzung Cannabidiol in einer Menge von 0,01-0,5 Gew.-% enthält, bezogen auf das Gesamtgewicht der Zusammensetzung.

8. Die Wundheilungszusammensetzung nach den Ansprüchen 1 bis 7, **dadurch gekennzeichnet, dass** die Zusammensetzung ethanolischen Extrakt von Hypericum perforatum in einer Menge von 0,1-1 Gew.-% enthält, bezogen auf das Gesamtgewicht der Zusammensetzung.

9. Die Wundheilungszusammensetzung nach den Ansprüchen 1 bis 8, **dadurch gekennzeichnet, dass** die Zusammensetzung ethanolischen Extrakt von Calendula officinalis in einer Menge von 0,1-1 Gew.-% enthält, bezogen auf das Gesamtgewicht der Zusammensetzung.

10. Die Wundheilzusammensetzung nach den Ansprüchen 1 bis 9, **dadurch gekennzeichnet, dass** die Zusammensetzung enthält:
• Schneckenschleim, 49,5 Gew.-%;
• Chitosan, 12,45 Gew.-%;
• Cannabidiol, 0,05 Gew.-%;
• Hypericum perforatum ethanolischer Extrakt, 0,5 Gew.-% und
• Calendula officinalis ethanolischer Extrakt, 0,5 Gew.-%, basierend auf dem Gesamtgewicht der Zusammensetzung.

11. Eine topische Arzneimittelformulierung zur Wundheilung, die die Wundheilungszusammensetzung nach den Ansprüchen 1-10 enthält.

12. Die topische Arzneimittelformulierung gemäß Anspruch 11, **dadurch gekennzeichnet, dass** die Formulierung in Form eines Films, einer Creme, eines Gels, einer Salbe oder einer Lotion vorliegt.

13. Verfahren zur Herstellung der Wundheilzusammensetzung nach den Ansprüchen 1-10, wobei das Verfahren die Schritte umfasst:
- extraktion von Schneckenschleim aus Schnecken;
- gefriertrocknung des im vorherigen Schritt erhaltenen Schneckenschleimextrakts für mindestens 24 Stunden bei einer Temperatur von -45 °C, um trockenes Schneckenschleimpulver zu erhalten;
- kombinieren des im vorherigen Schritt erhaltenen Schneckenschleims mit Cannabidiol, ethanolischem Extrakt aus Hypericum perforatum und ethanolischem Extrakt aus Calendula officinalis und mit einem Vernetzungsmittel; und
- einbetten in Mikropartikel auf Chitosanbasis, vorzugsweise durch Sprühtrocknung unter Verwendung einer Zweistoffdüse.

14. Die Wundheilungszusammensetzung nach den Ansprüchen 1 bis 10 zur Verwendung als Arzneimittel.

15. Die Wundheilungszusammensetzung nach den Anspruch 14 zur Verwendung bei der Behandlung von Hauterkrankungen oder Wunden wie Psoriasis, Ekzemen, atopischer Dermatitis, diabetischem Fußgeschwür.

## Revendications

1. Composition cicatrisante comprenant les composants suivants:
• Mucus d'escargot;
• Chitosane;
• Cannabidiol;
• Extrait éthanolique d'Hypericum perforatum et
• Extrait éthanolique de Calendula officinalis, **caractérisée en ce que** les composants sont incorporés avec un agent de réticulation dans des microparticules à base de chitosane.

2. Composition cicatrisante selon la revendication 1, **caractérisée en ce que** le mucus d'escargot est sous forme de poudre.

3. Composition cicatrisante selon la revendication 2, **caractérisée en ce que** la poudre de mucus d'escargot a une taille de particule de D50 4,7 µm±0,0035 avec une valeur Span de 1,48±0,006.

4. Composition cicatrisante selon les revendications 1 à 3, **caractérisée en ce que** le cannabidiol est sous forme d'huile de CBD.

5. Composition cicatrisante selon les revendications 1 à 4, **caractérisée en ce que** la composition contient du mucus d'escargot à raison de 10 à 50 % en poids, par rapport au poids total de la composition.

6. Composition cicatrisante selon les revendications 1 à 5, **caractérisée en ce que** la composition contient du chitosane à raison de 10 à 15 % en poids, par rapport au poids total de la composition.

7. Composition cicatrisante selon les revendications 1 à 6, **caractérisée en ce que** la composition contient du cannabidiol à raison de 0,01 à 0,5 % en poids, par rapport au poids total de la composition.

8. Composition cicatrisante selon les revendications 1 à 7, **caractérisée en ce que** la composition contient de l'extrait éthanolique d'Hypericum perforatum à raison de 0,1 à 1 % en poids, par rapport au poids total de la composition.

9. Composition cicatrisante selon les revendications 1 à 8, **caractérisée en ce que** la composition contient de l'extrait éthanolique de Calendula officinalis à raison de 0,1 à 1 % en poids, par rapport au poids total de la composition.

10. Composition cicatrisante selon les revendications 1 à 9, **caractérisée en ce que** la composition contient:
• du mucus d'escargot, 49,5 % en poids;
• du chitosane, 12,45 % en poids;
• du cannabidiol, 0,05 % en poids;
• de l'extrait éthanolique d'Hypericum perforatum, 0,5 % en poids et de l'extrait éthanolique de Calendula officinalis, 0,5 % en poids, par rapport au poids total de la composition.

11. Formulation de médicament topique pour la cicatrisation des plaies contenant la composition cicatrisante selon les revendications 1 à 10.

12. Formulation de médicament topique selon la revendication 11, **caractérisée en ce que** la formulation se présente sous la forme d'un film, d'une crème, d'un gel, d'une pommade ou d'une lotion.

13. Procédé de fabrication de la composition cicatrisante selon les revendications 1 à 10, le procédé comprenant les étapes suivantes:
- extraction de mucus d'escargot à partir d'escargots;
- lyophilisation de l'extrait de mucus d'escargot obtenu à l'étape précédente, pendant au moins 24 heures, à une température de -45°C pour obtenir une poudre de mucus d'escargot sèche;
- association du mucus d'escargot obtenu à l'étape précédente avec du cannabidiol, de l'extrait éthanolique d'Hypericum perforatum et de l'extrait éthanolique de Calendula officinalis et avec un agent de réticulation; et
- incorporation dans des microparticules à base de chitosane de préférence par un procédé de séchage par pulvérisation utilisant une buse à double fluide.

14. Composition cicatrisante selon les revendications 1 à 10 destinée à être utilisée comme médicament.

15. Composition cicatrisante selon la revendication 14 destinée à être utilisée dans le traitement d'une affection cutanée ou d'une plaie, telle que le psoriasis, l'eczéma, la dermatite atopique, l'ulcère du pied diabétique.
